# EUROPEAN PATENT SPECIFICATION

(11) **EP 1 308 726 B1**
(45) Date of publication and mention of the grant of the patent: **08.03.2006**
(21) Application number: 01402843.5
(22) Date of filing: 05.11.2001
(51) Int. Cl.: G01N 33/50, G01N 33/542

(54) **Methods and compositions for screening modulators of integrins**
Screeningverfahren für Modulatoren von Integrinen
Procédés et composés pour le criblage des modulateurs d'intégrines

(43) Date of publication of application: 07.05.2003
(73) Proprietor: Warner-Lambert Company LLC, Morris Plains, New Jersey 07950 (US)
(72) Inventor: Hamon, Jacques, 91530 - Saint-Maurice Montcourronne (FR); Normant, Emmanuel, 92160 - Antony (FR)
(74) Representative: Vanhee-Brossollet, Christine

(56) References cited:
- WO-A-99/58573
- US-A- 5 837 478
- US-B1- 6 210 914
- WALSH G M ET AL: "Integrin alpha-4-beta-7 mediates human eosinophil interaction with MAdCAM-1, VCAM-1 and fibronectin." IMMUNOLOGY, vol. 89, no. 1, 1996, pages 112-119, XP002200149 ISSN: 0019-2805
- DOYLE PAUL M ET AL: "Solution structure of a biologically active cyclic LDV peptide analogue containing a type II' beta-turn mimetic." INTERNATIONAL JOURNAL OF PEPTIDE & PROTEIN RESEARCH, vol. 47, no. 6, 1996, pages 427-436, XP001073789 ISSN: 0367-8377
- TIDSWELL M ET AL: "Structure-function analysis of the integrin beta 7 subunit: identification of domains involved in adhesion to MAdCAM-1." JOURNAL OF IMMUNOLOGY (BALTIMORE, MD.: 1950) UNITED STATES 1 AUG 1997, vol. 159, no. 3, 1 August 1997 (1997-08-01), pages 1497-1505, XP002200150 ISSN: 0022-1767

## Description

### Field of Invention

The present invention relates to methods of screening compounds that modulate the activity of integrins, more particularly integrins of the αβ7 sub-family. The invention is more preferably based on the SPA technology to screen compounds that modulate integrins of the αβ7 sub-family, more specifically the activity of α4β7 or αEβ7 integrins. The invention also includes compositions, products, kits for use in performing the above methods, as well as the compounds identified by said methods, and their uses.

### Background of the Invention

Integrins are a family of transmembrane adhesion molecules involved in cell-cell and cell-extracellular matrix communications. Integrins comprise various families of heterodimeric molecules, comprising an α and a β sub-unit, non-covalently expressed on the cell surface (Wardlaw, Drugs of the Future, 1999, 24(3), 279). The β7 integrin subunit can pair two α chains, α4 and αE , and is expressed mainly on lymphocytes. As an α4 heterodimer, it binds to the mucosal adressin cell adhesion molecule MADCAM-1 and mediates selective recruitment of memory T and B cells to gastrointestinal mucosa and gut associated lymphoid tissue. As an αE heterodimer, it binds to E-cadherin and mediates adhesion of intra epithelial T-lymphocytes to epithelial cells.

Expression of MADCAM-1 appears to be regulated at sites associated with inflammatory bowel disease (Briskin et al., 1997, AJP 151(1), 97). Administration of the anti- α4β7 monoclonal antibody ACT-1 to chronically colotic cotton-top tamarin monkeys inhibites recruitment of various leukocyte subclasses to inflammed colonic mucosa (Hesterberg et al., 1996, Gastroenterology 111, 1373). Reduction of colonic inflammatory activity in SCID mice reconstituted with CD45Rbhi CD4+ T cells has been observed after administration of antibodies to β7 and/or to MADCAM-1 (Picarella et al., 1997, J. Immunol. 158, 2099). Walsh et al., 1996, Immunology 89, 112-119, suggest α₄β₇-dependent bonding of eosinphils to VCAM-1 and MadCAM-1 transfectants. Altogether, these elements suggest that α4β7 represents an interesting target for certain inflammatory diseases, particularly inflammatory bowel diseases.

With respect to αEβ7, this integrin has, to our knowledge, never been used as a target for drug screening. However, the inventors of the present application have now obtained experimental results showing that this molecule could play a critical role in T cell recruitment and also represents an interesting target for various diseases such as, more specifically, asthma. In this regard, αE deficiency reduces airway hyper-responsiveness in Ag-challende mice and an anti-αE antibody (M290) has demonstrated good activity in a mouse model of ovalbumin-induced eosinophilia (unpublished results by inventors). Furthermore, αEβ7 may also be involved in inflammatory bowel diseases.

Accordingly, α4β7 and αEβ7 integrins represent an interesting target for the development of drugs or pharmacologically active compounds. In particular, compounds having the ability to modulate the activity of these integrins would represent high potential compounds for the treatment of all diseases where they are involved such as inflammatory diseases (e.g., inflammatory bowel disease, multiple sclerosis, psoriasis, epidermodysplasia verruciformis, inflammatory arthritis), allergic diseases (e.g., dermatitis, T helper-1 related diseases, chronic obstructive pulmonary disease, asthma, etc.) and immune diseases.

The availability of assays suitable to screen compounds having such property would thus be of major interest. In this respect, the current integrin assays are adhesion assays using ligand-coated plates contacted with cells expressing integrins and a test compound. However, this test is not suitable for high throughput format and lacks sensitivity and/or reproducibility. A scintillation proximity assay (SPA) has been used for the study of inhibitors of d₄ß₁; and VCAM-1 interactions (Doyle et al., 1996, Int. J.Peptide Protein Res. 47, 427-436). Accordingly, to our knowledge, no efficient assay has been reported in the art allowing an efficient, reliable, sensitive and reproducible screening of compounds that modulate the activity of integrins having a β7 subunit.

### Summary of the Invention

The present invention discloses compositions and methods for the screening of compounds that modulate, inhibit or activate the activity of α4β7 or αEβ7 integrins, with reliability and efficacy. The methods according to this invention are simple, reliable, sensitive, convenient and economical, and allow screening of compounds, on a high throughput basis. In particular, the invention can be used to screen, in parallel, large numbers of compounds, including combinatorial libraries of compounds, to identify drug candidates or targets. This type of invention thus allows, for the first time, to screen active compounds using α4β7 or αEβ7 integrins as targets, for the selection, improvement and/or development of therapeutically active products.

An object of this invention resides more specifically in a method of selecting or identifying a compound that modulates the activity of integrin α4β7 or αEβ7. The method more preferably comprises the identification, selection, characterization or screening of compounds that inhibit the activity of integrin α4β7 or αEβ7.

A particular object of the present invention resides in a method of selecting or identifying a compound that modulates, inhibits or activates the activity of a α4β7 or αEβ7 integrin, the method comprising:
- contacting (i) a labelled membrane preparation comprising α4β7 or αEβ7 integrin and a candidate compound with (ii) a support material that binds α4β7 or αEβ7 integrin, respectively, via a ligand thereof, and contains a scintillant, and
- assessing the amount of α4β7 or αEβ7 integrin bound to the support.

The support material may be composed of or comprise various elements, such as polymers, gels, glass, artificial or organic elements, etc. The support may be further functionalized, and may be shaped into various forms, including beads. Since the method preferably uses the scintillation proximity technology (SPA), the support material further comprises a scintillant, which can be excited upon binding of the labeled integrin thereto or when the labeled integrin material is brought to proximity thereof. In a preferred embodiment, the support material comprises a scintillant and is coated with a ligand of the integrin, i.e., with a compound that is able to (specifically) interact with integrin or a functional part thereof.

In the method of the present invention, determining the amount of integrin bound to the support material preferably comprises determining the scintillation signal of the support. More preferably, the method comprises comparing the amount of integrin bound to the support in the presence and in the absence of a candidate compound and/or in the presence of a blocking antibody, and identifying the compound that modulates said amount. Furthermore, the invention can be used to screen large amounts of candidate compounds in parallel, such as whole or part of combinatorial libraries of compounds.

The invention can be used for selecting, identifying, characterizing, improving, comparing, etc... compounds that modulate, inhibit or activate the activity of integrin α4β7 or αEβ7. The invention is more particularly suited for screening inhibitors of integrin α4β7 or αEβ7.

A further object of this invention resides in a kit for use in screening modulators of αEβ7 or α4β7 integrin, the kit comprising a labeled membrane preparation comprising αEβ7 or α4β7 integrin or a functional part thereof and/or a support material wherein the support material comprises a ligand that binds αEβ7 or α4β7 integrin or a functional part thereof and contains a scintillant.

### Legend to the Drawings

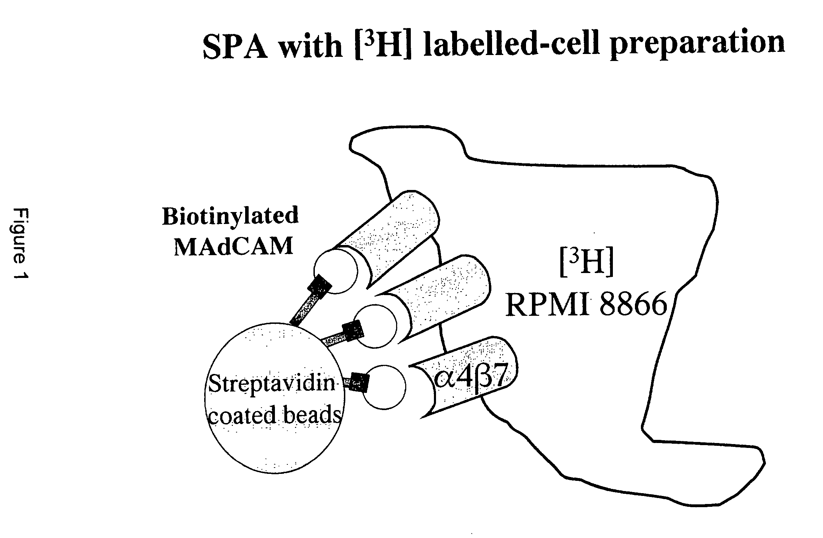

Figure 1 : Schematic representation of the SPA based assay

### Detailed Description of the Invention

As indicated, this invention resides, generally, in improved methods of screening for modulators of integrins. These methods, generally, use SPA format and membrane preparations comprising the selected integrin or functional parts thererof (see figure 1). The methods can be used to screen activators as well as inhibitors of integrins, e.g., compounds that increase or decrease the binding of integrins to ligands thereof. Preferably, inhibitors are selected, i.e., compounds that decrease the levels of said interaction, typically by at least 20%, preferably by at least 50%.

Typically, the present invention resides in a method of selecting or identifying a compound that modulates, inhibits or activates the activity of a α4β7 or αEβ7 integrin, the method comprising:
- contacting (i) a labelled membrane preparation comprising α4β7 or αEβ7 integrin and a candidate compound with (ii) a support material that binds α4β7 or αEβ7 integrin, respectively, and contains a scintillant, and
- assessing the amount of α4β7 or αEβ7 integrin bound to the support.

In a preferred embodiment, the amount of integrin bound to the support in the presence of a candidate compound is compared to the amount of integrin bound to the support in the absence of a candidate compound and/or in the presence of a blocking antibody, compounds modulating said amount representing compounds that modulate the activity of the integrin.

As indicated, the present invention preferably involves a scintillation assay such as the scintillation proximity assay (SPA). Scintillation proximity assay (SPA) technology involves the use of scintillant support material (e.g., beads) that contain an organic scintillant such as diphenyloxazole (PPO). Assays are usually carried out in aqueous buffers using radioisotopes such as ³H, ¹²⁵I, ¹⁴C, ³⁵S or ³³P that emit low-energy radiation, the energy of which is easily dissipated in an aqueous environment. For example, the electrons emitted by ³H have an average energy of only 6 keV and have a very short path length (-1 ~tm) in water. If a molecule labelled with one of these isotopes is bound to the surface of the support material, either directly or via interaction with another molecule previously coupled to the support material, the emitted radiation will activate the scintillant and produce light. The amount of light produced, which is proportional to the amount of labelled molecules bound to the support material, can be measured conveniently with a liquid scintillation (LS) counter. If the labelled molecule is not attached to the support material, its radiation energy is absorbed by the surrounding aqueous solvent before it reaches the support material, and no light is produced. Thus, bound ligands give a scintillation signal, but free ligands give a very low background, and the need for a time-consuming separation step, characteristic of conventional radioligand binding assays, is eliminated. The manipulations required in the assays are reduced to a few simple pipetting steps leading to better precision and reproducibility, and a higher throughput.

In a more preferred embodiment, the method comprises the use of SPA beads coated with a ligand of integrin, typically an endogenous ligand of integrin. The coating is preferably carried out through chemical or physical interaction with the beads, although other binding means can be contemplated. In particular, coating may be performed using streptavidin-beads and biotinylated ligand molecules.

This invention stems from the selection and validation of integrins as targets for development of drugs in inflammatory, allergic and immune diseases, as well as from the selection of efficient assay conditions to screen such drugs. In this regard, the invention unexpectedly shows that particular SPA assay formats using membrane preparations can be used to identify compounds that interfere with complex protein-protein interactions between complex molecules such as heterodimeric receptors, in particular recombinant heterodimeric receptors. The invention further shows that high throughput is feasible, since 384-wells plates format can be used, with low volumes.

### The support Material

The present invention now discloses a novel method of screening active compounds using SPA assay format and support material. The support material has the ability to bind the selected integrin complex in essentially native conformation, or functional parts thereof.

In this regard, the support may be composed, at least in part, of silicate, polyvinyltoluene (PVT), (poly)acrylamide, agarose, sepharose, polystyrene, etc. Specific examples of support material include PVT or silicate material, optionally coated with ligands such as WGA, streptavidin, polylysine, etc. More preferred material comprises yttrium silicate (YtSi) or PVT coated or functionalized, in particular coated with a ligand of the integrin.

In a more preferred embodiment, the support contains a scintillant (or an organic scintillant). The scintillant is preferably water insoluble and excitable to a higher energy state upon binding of the labeled integrin to the support. The scintillant should produce sufficient light energy to be detected using suitable device (scintillation counter, for instance). A typical example of scintillant is diphenyloxazole (PPO). This scintillant is efficiently excited by radioisotopes emitting beta rays.

Suitable support material for use in the present invention may be found in the commerce, such as for instance from Amersham products WGA-coated PVT beads (RPNQ0001), PEI-treated WGA PVT beads (RPNQ0003), streptavidin-coated PVT beads (RPNQ0007 ; RPNQ0007), polylysine-coated yttrium silicate beads (RPNQ0010), WGA coated yttrium silicate beads(RPNQ0011), streptavidin coated yttrium silicate beads(RPNQ0012) and RNA-binding yttrium silicate SPA beads (RPNQ0013).

Although the support material may be of various shapes, it is typically in the form of beads (in this regard, see "Proximity News", 39 (Dec 1998), 1). It should be understood that the precise shape and composition of the support material may be adjusted by the skilled person using common general knowledge.

In a preferred embodiment, the support material (e.g., the beads) is linked to (or coated with) a(n endogenous) ligand of α4β7 or αEβ7 integrin. The term ligand designate any molecule that has the ability to interact with the selected integrin. In a more preferred embodiment, the ligand is an endogenous ligand of the integrin, i.e., a compound that is known to physiologically interact with the selected integrin. Indeed, it is most preferred to use selective ligand molecules, i.e., molecules that interact preferentially with the desired integrin, and to use physiological ligand molecule to produce the most reliable results.

In this respect, in a first preferred variant, the method employs support material coated with (or linked to) MADCAM-1 or a functional part thereof. MADCAM-1 is an endogenous ligand of integrin α4β7. The nucleic and amino acid sequences of MADCAM-1 are available in Genbank, accession number MN_007164.

In a second preferred variant, the method employs support material coated with E-cadherin or a functional part thereof. E-cadherin is an endogenous ligand of integrin αEβ7. The nucleic and amino acid sequences of E-cadherin are available in Genbank, accession number XM_007840.

The term "functional part" designates any variant or fragment of the ligand that retains the ability to interact with the selected integrin. In particular, the funtional part may consist of a fragment of the ligand containing the binding site and devoid of cytoplasmic domain. The functional part may also comprise a variant of the ligand, having one or several amino acid modification(s) (e.g., deletion, substitution, mutation and/or addition), that still exhibits the ability to bind to the integrin. Such variants also include naturally-ocurring variants such as polymorphisms, splicing forms, homologs from different species, etc.

The ligand may be coated to the support material through different means, such as by covalent or non-covalent interactions. Preferably, the ligand is coated covalently, through a chemical linker or spacer, such as streptavidin/biotin, for instance.

Generally, from 0.05 to 5 mg of support material (e.g., coated beads) is used for each assay. It should be understood that the precise amount of support material can be adjusted by the skilled person, depending on the support material, amounts of reagents, etc.

### The labelled Membrane preparation

As indicated above, this invention employs a labelled membrane preparation comprising the selected integrin, whose activity is to be detected under the conditions described above. The inventors indeed showed that the screening is much more efficient and reliable when membrane preparations are used, instead of isolated or purified integrin polypeptide. Furthermore, the inventors have now shown that membrane preparations can be used in the screening to assess the interaction between a complex receptor molecules and the ligand in a high throughput format.

The membrane preparation may be a whole cell or a preparation derived from a whole cell comprising a membrane fraction thereof, wherein said cell or membrane fraction comprise α4β7 or αEβ7 integrin or a functional part thereof. The membrane preparation may also be an artificial membrane preparation comprising the selected integrin, e.g., a micelle or a liposome produced by mixing lipids and the integrin polypeptides together under suitable conditions. In a more preferred embodiment, the membrane preparation is a cell lysate produced from a cell expressing the selected integrin polypeptide or a functional part thereof.

In a particular embodiment, the method uses a membrane preparation, such as a cell lysate or a cell fraction, comprising the integrin, derived (or obtained) from (recombinant) mammalian or bacterial cells.

The integrin may be of various origin, such as human or animal, preferably human. The integrin may be a naturally-occurring integrin, e.g., prepared from a culture of cells that naturally produce said molecule (tissue culture, cell culture, etc.), or a recombinant molecule, prepared from cells containing a recombinant nucleic acid encoding the same. In this respect, the integrin may be obtained from transfected cells containing a nucleic acid encoding said molecule. The nucleic acid sequences encoding a human integrin αEβ7 and α4β7 have been cloned and described in the art. For instance, these sequences are available at Genbank, under accession numbers XM_029724; XP_039011; XP_008508; XM_039011.1; XM_008508.5. The sequence may be transfected into cells, using various plasmids and/or vectors, containing various promoters, to produce the recombinant molecule. The lysate of such cells (or other preparations derived therefrom) may be used in the screening assays of this invention. The cells may be of various origin, such as mammalian, eukaryotic (e.g., yeasts), prokaryotic (e.g., bacteria), vegetal, etc. Usually, preferred cells are of mammalian origin, so that the recombinant integrin is functional and processed according to natural mechanisms. However, non-human cells may be used to further increase the selectivity of the method, i.e., to produce cell membranes expressing the selected human integrin and no other human integrin polypeptide. In this regard, preferred mammalian or human cells are cells that essentially do not naturally express integrin molecules.

The cells may be primary cells or established cell cultures. They may be cultured in any appropriate medium, and then treated to prepare the integrin-containing membrane preparation (cell extracts, fractions, and the like). Typically, the cells are subjected to physical and/or chemical treatment, to produce the integrin-containing material. In a typical experiment, the cells are subjected to enzymatic and/or chemical and/or physical lysis, preferably using trypsin, thaw/freeze cycle(s), ultra-sounds, etc., either alone or in various combinations. The cell extracts (or fractions) are collected, and may be further concentrated, suspended in appropriate buffers, purified, conditioned, etc.

Typically, the integrin membrane preparation used is a cell lysate or fractions (or other material derived from cells) comprising. The integrin membrane preparation may also comprise total (transfected) cell extracts.

In a preferred embodiment, the membrane preparation is obtained from a recombinant (mammalian) cell expressing a α4β7 or αEβ7 integrin polypeptide molecule, even more preferably from a recombinant cell that expresses human α4β7 or αEβ7 integrin, or a functional part thereof. To produce such membrane preparation, various cells may be used, such as cells that naturally express the selected integrin, or cells transduced with a nucleic acid molecule encoding the same or a functional part thereof. The term functional part is as defined above for the ligand. Indeed, it is not necessary that the expressed integrin is identical to the naturally-occuring integrin, as long as (i) it retains a functional binding site and (ii) it is able to interact with the ligand coated on the support material. Accordingly, the integrin may lack cytoplasmic domains or amino acids and/or contain amino acid variations as compared to the wild-type molecules.

As indicated, the membrane preparation is labelled to allow detection of an interaction with the coated support material. The labelled membrane preparation is preferably radiolabelled. Radiolabeling can be performed using various radioisotopes, including ³H, ¹²⁵I, ¹⁴C, ³⁵S, ³³P or ³²P. Preferably, the radioisotope should emit low-energy radiation, the energy of which is easily dissipated in an aqueous environment. Indeed, it is required that unbound labeled membranes essentially fail to activate the scintillant contained in the support material. The nature of the isotope may be selected also depending on the type of scintillant. For instance, where PPO is used as a scintillant, the isotope should preferably emit beta rays, such as ³H for instance. In a preferred embodiment, the membrane preparations are tritiated.

Labelling may be performed according to various techniques known in the art, such as by incorporating labelled elements into the cell membranes (e.g., labelled amino acids, lipids, etc.), or by contacting the membrane preparations with a label (labelled ligand, etc.). Preferably, the membranes are labelled by incorporation of labelled elements during cell culture. Even more preferably, they are labelled by incorporation of labelled amino acids. Accordingly, in a typical embodiment, the membrane preparation is radiolabelled by culturing a cell in the presence of a radiolabeled amino acid prior to preparing the membrane preparation. The labelled amino acid may be for instance methionine, which is incorporated in most polypeptides, since it is encoded by the start ATG codon. It should be understood that other labelled amino acids may be used.

The amount of labeled membrane preparation used for the assay can be adjusted by the skilled person. In a typical experiment, between 1X10⁴ to 5X10⁸ cpm/ml of radiolabelled membrane preparation is used for each assay reaction, even more preferably between 1X10⁵ to 5X10⁷ cpm/ml. In a typical experiment, between 1X10⁶ to 5X10⁶ cpm/ml are used.

### The assay conditions

The assay can be performed in any appropriate support or device, including plate, tube, flask, and the like. Generally, contacting is performed in multi-well plates, allowing multiple assays to be carried out in parallel. Typical supports include microtiter plates, especially the 96-well or 384-well and higher throughput microtiter plate formats, which are easy to manage and easy to illuminate with conventional excitation. Other formats may also be used, including larger microtiter plates or nanotechnologies.

Depending on the support and test compound, varying amounts of reagents can be used in the assay. Typically, the following amounts may be distributed in a final maximum volume of 250 µl per well:
- 0.05-5 mg/ml of ligand-coated beads, and
- 1X10⁴ to 5X10⁸ cpm/ml of radiolabelled membrane preparation.
   The reaction may be carried out in any suitable assay buffer, comprising for instance any buffer, saline solution, aqueous solution, etc., that allows contacting of the various reagents and does not alter their biological activity.

It should be understood that the precise respective amounts (or concentration) of reagents and test compounds can be adjusted by the user, depending on the type of compound, the type of integrin, the length of incubation period, etc. Furthermore, if necessary, the reagents can be mixed in the presence of additional agents to improve the performance of the assay.

The contacting in step i) can last for up to 6 hours, typically less than 4 hours. Indeed, the various reagents are preferably incubated for a period of time sufficient to allow an interaction to occur. Depending on the assays, this period usually lasts less than about 3 hours. In a typical experiment, the mixing is performed for about 1 hour or less. The support may be added for about 10 minutes to several hours. Typically, the reaction mixture is shaken during the first 5-30 minutes, and the mixture is left for an other 15-100 minutes, or longer.

The amount or quantity of integrin bound to the support can be assessed by various ways, as an indication of the activity of the candidate compound. Generally, it is assessed by scintillation counting using conventional devices. In particular, the counts can be measured directly in the reaction mixture, with no need for any separation step. Alternatively, the support bound lipid may be detected or quantified using other conventional techniques, such as chromatography, immuno-assay,etc.

A preferred embodiment of this invention includes a method of selecting or identifying a compound that modulates the activity of integrin α4β7, comprising :
- contacting, in the presence or absence of a candidate compound, (i) a labelled membrane preparation comprising α4β7 integrin or a functional part thereof with (ii) a support material coated with MADCAM-1 or a functional part thereof that selectively binds α4β7 contained in said membrane preparation, said support material comprising a scintillant, and
- assessing the effect of the candidate compound on the activity of integrin α4β7 by comparing the scintillation signal in the presence and absence of the candidate compound and/or in the presence of a blocking antibody, compounds that modulate said scintillation signal representing compounds that modulate said activity.

An other preferred embodiment of this invention resides in a method of selecting or identifying a compound that modulates the activity of integrin αEβ7, comprising :
- contacting, in the presence or absence of a candidate compound, (i) a labelled membrane preparation comprising αEβ7 integrin or a functional part thereof with (ii) a support material coated with E-Cadherin or a functional part thereof that selectively binds αEβ7 contained in said membrane preparation, said support material comprising a scintillant, and
- assessing the effect of the candidate compound on the activity of integrin αEβ7 by comparing the scintillation signal in the presence and absence of the candidate compound and/or in the presence of a blocking antibody, compounds that modulate said scintillation signal representing compounds that modulate said activity.

The above methods are preferably directed at identifying or screening integrin inhibitors.

### The test (or candidate) compound(s)

The test compound can be any product in isolated form or in mixture with any other material (e.g., any other product(s)). The compound may be defined in terms of structure and/or composition, or it may be undefined. For instance, the compound may be an isolated and structurally-defined product, an isolated product of unknown structure, a mixture of several known and characterized products or an undefined composition comprising one or several products. Examples of such undefined compositions include for instance tissue samples, biological fluids, cell extracts, vegetal preparations, etc. The test compound may be any organic or inorganic product, including a polypeptide (or a protein or peptide), a nucleic acid, a lipid, a polysaccharide, a chemical product, or any mixture or derivatives thereof. The compounds may be of natural origin, synthetic origin, including libraries of compounds. As will be further discussed below, the present invention is particularly adapted for the screening of large numbers of compounds, such as combinatorial libraries of compounds. Indeed, the instant invention provides compositions and methods allowing efficient and simple screening of several compounds in short periods of time. In particular, the instant methods can be partially automated, thereby allowing efficient and simultaneous screening of large sets of compounds.

Generally, the activity of the test compound(s) is unknown, and the method of this invention is used to identify compounds exhibiting the selected property (e.g, integrin modulators). However, in particular instances where the activity (or type of activity) of the test compound(s) is known or expected, the method can be used to further characterize said activity (in terms of specificity, efficacy, etc.) and/or to optimise said activity, by assaying derivatives of said test compounds.

The invention also includes kits for use in screening modulators of αEβ7 or α4β7 integrin, the kit comprising a labeled membrane preparation comprising αEβ7 or α4β7 integrin or a functional part thereof and/or a support material wherein the support material binds αEβ7 or α4β7 integrin or a functional part thereof and contains a scintillant. The kit may further include the reagents and/or protocols for SPA technology, such as buffers, etc.

### EXAMPLES

### 1. αEβ7 SPA assay

### 1.1. Material:

Transfectants of the human chronic myelogenous leukemia cell line K562 that express αEβ7, and proteins such as E-Cadherin-Fc Fusion Protein, were as disclosed in Brenner et al. (*Molecular basis for Leukocyte Integrin αEβ7 Adhesion to Epithelial (E) - Cadherin, «Structure-Function analysis of integrin β7subunit*). These may be prepared according to known techniques. Biotinylation of (E)-Cadherin was performed according to classical protocol (*Cf : Amersham proximity news : Biotinylation techniques*...). Growth medium including all supplements were purchased from LifeTech (Paris, France) [³H]methionine (TRK583, 85 Ci/mmol) and streptavidin SPA beads (RPNQ 0007) were purchased from Amersham (Paris, France).

### 1.2. Culture

K562 are grown in a RPMI 1640 with L-glutamine medium (Life Tech #21875-0.34), Fetal Clone 110%, Additionnal L-Glu 2 mM, Pen / Strep 100 X , Geneticin 550 mg/L , Hygromycin B 550 mg/L. Cells are split every 2- 3 days. We usually resuspend at 0.4 million / ml concentration in COSTAR 150 cm² flasks (standing down).

### 1.3. Membrane tritiation

K562 human cell line (50.10⁶ cells/100 ml) are grown for 18hrs in a methionin free RPMI1640 (LifeTech #11876-026) with 10 % FBS in presence of 80µCi [³H]methionine (Amersham #TRK583, 85 Ci/mmol). Cells are washed twice with 25 ml PBS, centrifuged down at 1500 rpm for 10 min and resuspended in 10 ml for 50.10⁶ cells of a hypotonic lysis buffer (20mM Hepes, 2mM Cacl₂, 20mM NaCl and 25ug/ml aprotinin) and kept at -80 °C.

### 1.4. Scintillation proximity assay

Streptavidin SPA beads are mixed with biot-E-Cadherin (5µg/mg beads) for 30 minutes at room temp in assay buffer (HBSS (Gibco, 14025-076), 0.5 mM Mn²⁺ (MnCl₂₎, 25 mM HEPES, pen/strep [1X], Complete™ EDTA-free (Protease inhibitor) : one tablet /50ml assay buffer (Boehringer Mannheim #1873580), 15 % glycerol), centrifuged down at 3000 rpm for 5 minutes and resuspended in the assay buffer at 70µg/100µl/well.
The scintillation proximity assay is carried out at room temperature in assay buffer. Each assay is performed in a 200 µl reaction volume containing 0.7 mg/ml SPA coated beads and 2.3. 10⁶ cpm/ml of tritiated membranes. Non specific signal is determined in the presence of high concentration of blocking antibody. The microtiter plates are incubated at room temperature for 45 minutes on an orbital shaker and counted in a Wallac Microbeta™ scintillation counter.

### 1.5. Results

The screening of several libraries has been completed. Several chemical entities have been selected and confirmed for their ability to inhibit integrin activity.

### 2. α4β7 SPA assay

### 2.1. Material

Cells expressing α4β7 : RPMI8866.
Ligand: MADCAM-Fc Fusion Protein
Biotinylation of MADCAM-Fc Fusion Protein was performed according to classical protocol (Cf : Amersham proximity news : Biotinylation techniques...).
Growth medium including all supplements were purchased from LifeTech (Paris, France). [³H]methionine (TRK583, 85 Ci/mmol) and streptavidin SPA beads (RPNQ 0007) were purchased from Amersham (Paris, France).

### 2.2. Culture

RPMI 8866 are grown in a RPMI 1640 with L-glutamine medium (Life Tech #21875-0.34), Gentamycin 0.1 mg/ml , FBS 10%. Cells are split every 2- 3 days. We usually resuspend at 0.4 million / ml concentration.

### 2.3. Membrane tritiation

RPMI 8866 human cell line (25.106 cells/50 ml) are grown for 18hrs in a methionin free RPMI1640 (LifeTech #11876-026) + 10 % FBS, gentamycin in presence of 80µCi [3H]methionine. Cells are washed twice with 25 ml PBS, centrifuged down at 1500 rpm for 10 min, resuspended in 10 ml for 50.106 cells of a hypotonic lysis buffer (20mM Hepes, 2mM Cacl2, 20mM NaCl and 25ug/ml aprotinin) and kept at -80 °C.

### 2.4. Scintillation proximity assay

Streptavidin SPA beads are mixed with biot-MADCAM (12µg/mg beads) for 30 minutes at room temp in assay buffer (HBSS, 0.5 mM Mn2+ (MnCl2), 25 mM HEPES, pen/strep [1X], Complete TM EDTA-free (Protease inhibitor) : one tablet / 50ml assay buffer (Boehringer Mannheim #1873580), 15 % glycerol), centrifuged down at 3000 rpm for 5 minutes and resuspended in the assay buffer at 70µg/100µl/well.
The scintillation proximity assay is carried out at room temperature in assay buffer. Each assay is performed in a 200 µl reaction volume containing 0.7 mg/ml SPA coated beads and 1.25 10⁶ cpm/ml of tritiated membranes. Non specific signal is determined in the presence of high concentration of blocking antibody. The microtiter plates are incubated at room temperature for 45 minutes on an orbital shaker and counted in a Wallac MicrobetaTM scintillation counter.

In conclusion, according to the preceeding examples a α4β7 SPA test has been set up, using tritiated α4β7 and MADCAM-1 beads. This test is very robust, both on 96 and 384 wells format, and allows us to screen quickly compounds libraries to sort out inhibitors of MADCAM-1 binding pocket with an exquisite sensitivity. This test doesn't require purified integrins, but instead, is run using raw extracts of (transiently transfected) cells. To our knowledge, this is the first efficient assay described so far for integrins having a β7 subunit, which opens new possibilities for the development of active molecules. Active molecules thus isolated or obtained may be used as drug candidates, or engaged in further optimization programs, to design oriented libraries or introduce further chemical modifications.

## Claims

1. A method of selecting or identifying a compound that modulates, inhibits or activates the activity of a α4β7 or αEβ7 integrin, comprising:
- contacting (i) a labelled membrane preparation comprising α4β7 or αEβ7 integrin and a candidate compound with (ii) a support material that binds α4β7 or αEβ7 integrin, respectively, via a ligand thereof, and contains a scintillant,
- assessing the amount of α4β7 or αEβ7 integrin bound to the support, and
- comparing the amount of integrin bound to the support in the presence and in the absence of the candidate compound, and/or in the presence of a blocking antibody, and selecting or identifying the compound that modulates said amount.

2. The method of claim 1 to select or identify an inhibitor of α4β7 integrin.

3. The method of claim 1 to select or identify an inhibitor of αEβ7 integrin.

4. The method of any one of the preceding claims, wherein the membrane preparation is a whole cell or a preparation derived from a whole cell comprising a membrane fraction thereof, wherein said cell or membrane fraction comprise α4β7 or αEβ7 integrin or a functional part thereof.

5. The method of claim 4, wherein the membrane preparation is a cell lysate.

6. The method of any one of claims 1 to 5, wherein the membrane preparation is obtained from a recombinant cell expressing α4β7 or αEβ7 integrin or a functional part thereof.

7. The method of claim 6, wherein the recombinant cell expresses human α4β7 or αEβ7 integrin, or a functional part thereof.

8. The method according to any one of the preceding claim, wherein the membrane preparation is radiolabelled, more preferably tritiated.

9. The method of claim 8, wherein the membrane preparation is radiolabelled by culturing a cell in the presence of a radiolabeled amino acid prior to preparing the membrane preparation.

10. The method of any one of the preceding claims , wherein the support material comprises yttrium-silicate, polyvinyltoluene (PVT), (poly)acrylamide, agarose, sepharose or polystyrene.

11. The method of claim 1 or 10, wherein the support is a bead.

12. The method of any one of the preceding claims; wherein the support material is coated with a ligand of α4β7 or αEβ7 integrin.

13. The method of claim 12, wherein the support is coated with MADCAM-1 or a functional part thereof.

14. The method of claim 12, wherein the support is coated with E-cadherin or a functional part thereof.

15. The method of any one of the preceding claims, wherein the scintillant is diphenyloxazole.

16. The method of claim 1, comprising contacting the candidate compound in an assay buffer with:
- 0.05-5 mg/ml of ligand-coated beads, and
- 1X10⁴ to 5X10⁸ cpm/ml of radiolabelled membrane preparation.

17. The method of any one of the preceding claims, wherein determining the amount of integrin bound to the support material comprises determining the scintillation signal of the support.

18. The method of claim 1, wherein several candidate compounds are tested in parallel.

19. The method of claim 1, wherein the contacting step is performed in a microtitration plate.

20. A method of claim 1 for selecting or identifying a compound that modulates the activity of integrin α4β7, comprising:
- contacting, in the presence or absence of a candidate compound, (i) a labelled membrane preparation comprising α4β7 integrin or a functional part thereof with (ii) a support material coated with MADCAM-1 or a functional part thereof that selectively binds α4β7 contained in said membrane preparation, said support material comprising a scintillant, and
- assessing the effect of the candidate compound on the activity of integrin α4β7 by comparing the scintillation signal in the presence and absence of the candidate compound and/or in the presence of a blocking antibody, compounds that modulate said scintillation signal representing compounds that modulate said activity.

21. A method of claim 1 for selecting or identifying a compound that modulates the activity of integrin αEβ7, comprising :
- contacting, in the presence or absence of a candidate compound, (i) a labelled membrane preparation comprising αEβ7 integrin or a functional part thereof with (ii) a support material coated with E-Cadherin or a functional part thereof that selectively binds αEβ7 contained in said membrane preparation, said support material comprising a scintillant, and
- assessing the effect of the candidate compound on the activity of integrin αEβ7 by comparing the scintillation signal in the presence and absence of the candidate compound and/or in the presence of a blocking antibody, compounds that modulate said scintillation signal representing compounds that modulate said activity.

22. A kit for use in screening modulators of αEβ7 or α4β7 integrin, the kit comprising a labeled membrane preparation comprising αEβ7 or α4β7 integrin or a functional part thereof and a support material wherein the support material comprises a ligand that binds αEβ7 or α4β7 integrin or a functional part thereof and contains a scintillant.

## Revendications

1. Procédé de sélection ou d'identification d'un composé qui module, inhibe ou active l'activité d'une intégrine α4β7 ou αEβ7, comprenant :
- la mise en contact (i) d'une préparation membranaire marquée comprenant l'intégrine α4β7 ou αEβ7 et un composé candidat et (ii) d'un matériau de support qui se lie à l'intégrine α4β7 ou αEβ7, respectivement, par le biais d'un ligand de celle-ci, et contient un scintillant,
- l'évaluation de la quantité d'intégrine α4β7 ou αEβ7 liée au support, et
- la comparaison de la quantité d'intégrine liée au support en présence et en l'absence du composé candidat et/ou en présence d'un anticorps bloquant, et la sélection ou l'identification du composé qui module ladite quantité.

2. Procédé selon la revendication 1 pour sélectionner ou identifier un inhibiteur de l'intégrine α4β7.

3. Procédé selon la revendication 1 pour sélectionner ou identifier un inhibiteur de l'intégrine αEβ7.

4. Procédé selon l'une quelconque des revendications précédentes, dans lequel la préparation membranaire est une cellule entière ou une préparation dérivée d'une cellule entière comprenant une fraction membranaire de celle-ci, ladite cellule ou fraction membranaire comprenant l'intégrine α4β7 ou αEβ7 ou une partie fonctionnelle de celle-ci.

5. Procédé selon la revendication 4, dans lequel la préparation membranaire est un lysat cellulaire.

6. Procédé selon l'une quelconque des revendications 1 à 5, dans lequel la préparation membranaire est obtenue à partir d'une cellule recombinante exprimant l'intégrine α4β7 ou αEβ7 ou une partie fonctionnelle de celle-ci.

7. Procédé selon la revendication 6, dans lequel la cellule recombinante exprime l'intégrine α4β7 ou αEβ7 humaine ou une partie fonctionnelle de celle-ci.

8. Procédé selon l'une quelconque des revendications précédentes, dans lequel la préparation membranaire est radiomarquée, plus préférentiellement tritiée.

9. Procédé selon la revendication 8, dans lequel la préparation membranaire est radiomarquée en cultivant une cellule en présence d'un acide aminé radiomarqué avant de préparer la préparation membranaire.

10. Procédé selon l'une quelconque des revendications précédentes, dans lequel le matériau de support comprend du silicate d'yttrium, du polyvinyltoluène (PVT), du (poly)acrylamide, de l'agarose, de la sépharose ou du polystyrène.

11. Procédé selon la revendication 1 ou 10, dans lequel le support est une bille.

12. Procédé selon l'une quelconque des revendications précédentes, dans lequel le matériau de support est enrobé avec un ligand de l'intégrine α4β7 ou αEβ7.

13. Procédé selon la revendication 12, dans lequel le support est enrobé avec la MADCAM-1 ou une partie fonctionnelle de celle-ci.

14. Procédé selon la revendication 12, dans lequel le support est enrobé avec la E-cadhérine ou une partie fonctionnelle de celle-ci.

15. Procédé selon l'une quelconque des revendications précédentes, dans lequel le scintillant est le diphényloxazole.

16. Procédé selon la revendication 1, comprenant la mise en contact du composé candidat dans un tampon de dosage avec :
- 0,05-5 mg/ml de billes enrobées de ligand, et
- 1.10⁴ à 5.10⁸ cpm/ml de préparation membranaire radiomarquée.

17. Procédé selon l'une quelconque des revendications précédentes, dans lequel la détermination de la quantité d'intégrine fixée au matériau de support comprend la détermination du signal de scintillation du support.

18. Procédé selon la revendication 1, dans lequel plusieurs composés candidats sont testés en parallèle.

19. Procédé selon la revendication 1, dans lequel l'étape de mise en contact est effectuée sur une plaque de microtitration.

20. Procédé selon la revendication 1 pour sélectionner ou identifier un composé qui module l'activité de l'intégrine α4β7, comprenant :
- la mise en contact, en présence ou en l'absence d'un composé candidat, (i) d'une préparation membranaire marquée comprenant l'intégrine α4β7 ou une partie fonctionnelle de celle-ci et (ii) d'un matériau de support enrobé de MACDAM-1 ou d'une partie fonctionnelle de celle-ci qui se lie sélectivement à l'α4β7 contenue dans ladite préparation membranaire, ledit matériau de support comprenant un scintillant, et
- l'évaluation de l'effet du composé candidat sur l'activité de l'intégrine α4β7 par comparaison du signal de scintillation en présence et en l'absence du composé candidat et/ou en présence d'un anticorps bloquant, les composés qui modulent ledit signal de scintillation représentant des composés qui modulent ladite activité.

21. Procédé selon la revendication 1 pour sélectionner ou identifier un composé qui module l'activité de l'intégrine αEβ7, comprenant :
- la mise en contact, en présence ou en l'absence d'un composé candidat, (i) d'une préparation membranaire marquée comprenant l'intégrine αEβ7 ou une partie fonctionnelle de celle-ci et (ii) d'un matériau de support enrobé de E-cadhérine ou d'une partie fonctionnelle de celle-ci qui se lie sélectivement à l'αEβ7 contenue dans ladite préparation membranaire, ledit matériau de support comprenant un scintillant, et
- l'évaluation de l'effet du composé candidat sur l'activité de l'intégrine αEβ7 par comparaison du signal de scintillation en présence et en l'absence du composé candidat et/ou en présence d'un anticorps bloquant, les composés qui modulent ledit signal de scintillation représentant des composés qui modulent ladite activité.

22. Kit à utiliser pour le criblage de modulateurs de l'intégrine αEβ7 ou α4β7, le kit comportant une préparation membranaire marquée comprenant l'intégrine αEβ7 ou α4β7 ou une partie fonctionnelle de celle-ci et un matériau de support, le matériau de support comprenant un ligand qui fixe l'intégrine αEβ7 ou α4β7 ou une partie fonctionnelle de celle-ci et contenant un scintillant.

## Patentansprüche

1. Verfahren zum Selektieren oder Identifizieren einer Verbindung, die die Aktivität eines α4β7- oder αEβ7-Integrins moduliert, inhibiert oder aktiviert, umfassend:
- Kontaktieren (i) einer markierten Membranpräparation, die α4β7- oder αEβ7-Integrin umfasst, und einer Kandidatenverbindung mit (ii) einem Trägermaterial, das α4β7- bzw. αEβ7-Integrin über einen Liganden davon bindet, und ein Szintillationsmittel enthält,
- Feststellen der Menge an α4β7- oder αEβ7-Integrin, das an den Träger gebunden ist, und
- Vergleichen der Menge an Integrin, das an den Träger in Anwesenheit und in Abwesenheit der Kandidatenverbindung und/oder in Gegenwart eines blockierenden Antikörpers gebunden ist, und Selektieren oder Identifizieren der Verbindung, die die besagte Menge moduliert.

2. Verfahren nach Anspruch 1, um einen Inhibitor von α4β7-Integrin zu selektieren oder identifizieren.

3. Verfahren nach Anspruch 1, um einen Inhibitor von αEβ7-Integrin zu selektieren oder zu identifizieren.

4. Verfahren nach irgendeinem der vorangehenden Ansprüche, wobei die Membranpräparation eine ganze Zelle oder eine Präparation, abgeleitet von einer ganzen Zelle, die eine Membranfraktion davon umfasst, ist, wobei die Zelle oder Membranfraktion α4β7- oder αEβ7-Integrin oder einen funktionellen Teil davon umfasst.

5. Verfahren nach Anspruch 4, wobei die Membranpräparation ein Zelllysat ist.

6. Verfahren nach irgendeinem der Ansprüche 1 bis 5, wobei die Membranpräparation von einer rekombinanten Zelle, die α4β7- oder αEβ7-Integrin oder einen funktionellen Teil davon exprimiert, erhalten ist.

7. Verfahren nach Anspruch 6, wobei die rekombinante Zelle menschliches α4β7- oder αEβ7-Integrin, oder einen funktionellen Teil davon, exprimiert.

8. Verfahren nach irgendeinem der vorangehenden Ansprüche, wobei die Membranpräparation radiomarkiert, stärker bevorzugt tritiiert, ist.

9. Verfahren nach Anspruch 8, wobei die Membranpräparation durch Kultivieren einer Zelle in Gegenwart einer radiomarkierten Aminosäure vor dem Herstellen der Membranpräparation radiomarkiert wird.

10. Verfahren nach irgendeinem der vorangehenden Ansprüche wobei das Trägermaterial Yttrium-Silikat, Polyvinyltoluol (PVT), (Poly)acrylamid, Agarose, Sepharose oder Polystyrol umfasst.

11. Verfahren nach Anspruch 1 oder 10, wobei der Träger ein Kügelchen ist.

12. Verfahren nach irgendeinem der vorangehenden Ansprüche 1, wobei das Trägermaterial mit einem Liganden von α4β7- oder αEβ7-Integrin beschichtet ist.

13. Verfahren nach Anspruch 12, wobei der Träger mit MADCAM-1 oder einem funktionellen Teil davon beschichtet ist.

14. Verfahren nach Anspruch 12, wobei der Träger mit E-Cadherin oder einem funktionellen Teil davon beschichtet ist.

15. Verfahren nach irgendeinem der vorangehenden Ansprüche, wobei das Szintillationsmittel Diphenyloxazol ist.

16. Verfahren nach Anspruch 1, das das Kontaktieren der Kandidatenverbindung in einem Assay-Puffer mit
- 0,05-5 mg/ml Liganden-beschichteten Kügelchen und
- 1X10⁴-5X10⁸ cpm/ml radiomarkierter Membranpräparation umfasst.

17. Verfahren nach irgendeinem der vorangehenden Ansprüche, wobei das Bestimmen der Menge des an das Trägermaterial gebundenen Integrins das Bestimmen des Szintillationssignals des Trägers umfasst.

18. Verfahren nach Anspruch 1, wobei verschiedene Kandidatenverbindungen parallel getestet werden.

19. Verfahren nach Anspruch 1, wobei der Schritt des Kontaktierens in einer Mikrotiterplatte durchgeführt wird.

20. Verfahren nach Anspruch 1 zum Selektieren oder Identifizieren einer Verbindung, die die Aktivität von Integrin α4β7 moduliert, umfassend:
- Kontaktieren, in Anwesenheit oder Abwesenheit einer Kandidatenverbindung, (i) einer markierten Membranpräparation, die α4β7-Integrin oder einen funktionellen Teil davon umfasst, mit (ii) einem Trägermaterial, beschichtet mit MADCAM-1 oder einem funktionellen Teil davon, das/der selektiv in der Membranpräparation enthaltenes α4β7 bindet, wobei das Trägermaterial ein Szintillationsmittel umfasst, und
- Feststellen des Effekts der Kandidatenverbindung auf die Aktivität von Integrin α4β7 durch Vergleichen des Szintillationssignals in Anwesenheit und Abwesenheit der Kandidatenverbindung und/oder in Gegenwart eines blockierenden Antikörpers, wobei Verbindungen, die das Szintillationssignal modulieren, Verbindungen darstellen, die die Aktivität modulieren.

21. Verfahren nach Anspruch 1 zum Selektieren oder Identifizieren einer Verbindung, die die Aktivität von Integrin αEβ7 moduliert, das umfasst:
- Kontaktieren, in Anwesenheit oder Abwesenheit einer Kanndidatenverbindung, (i) einer markierten Membranpräparation, die αEβ7-Integrin oder einen funktionellen Teil davon umfasst mit (ii) einem Trägermaterial, beschichtet mit E-Cadherin oder einem funktionellen Teil davon, das/der selektiv in der Membranpräparation enthaltenes αEβ7 bindet, wobei das Trägermaterial ein Szintillationsmittel umfasst, und
- Feststellen des Effekts der Kandidatenverbindung auf die Aktivität von Integrin αEβ7 durch Vergleichen des Szintillationssignals in Anwesenheit und Abwesenheit der Kandidatenverbindung und/oder in Gegenwart eines blockierenden Antikörpers, wobei Verbindungen, die das Szintillationssignal modulieren, Verbindungen darstellen, die die Aktivität modulieren.

22. Kit zur Verwendung beim Durchmustern von Modulatoren von αEβ7- oder α4β7-Integrin, wobei der Kit eine markierte Membranpräparation, die αEβ7- oder α4β7-Integrin oder einen funktionellen Teil davon umfasst, und ein Trägermaterial umfasst, wobei das Trägermaterial einen Liganden umfasst, der αEβ7-oder α4β7-Integrin oder einen funktionellen Teil davon bindet und ein Szintillationsmittel enthält.
